# EUROPÄISCHE PATENTSCHRIFT

(11) **EP 0 833 813 B1**
(45) Veröffentlichungstag und Bekanntmachung des Hinweises auf die Patenterteilung: **03.05.2000**
(21) Anmeldenummer: 96916160.3
(22) Anmeldetag: 24.05.1996
(51) Int. Cl.: C07C 215/14

(54) **KONZENTRIERTE LÖSUNGEN VON POLYHYDROXYALKYLAMINEN UND DEREN VERWENDUNG, INSBESONDERE ZUR HERSTELLUNG VON POLYHYDROXYALKYLCARBONSÄUREAMIDEN**
CONCENTRATED SOLUTIONS OF POLYHYDROXY ALKYL AMINES AND THEIR USE, ESPECIALLY FOR PRODUCING POLYHYDROXY ALKYL CARBOXYLIC ACID AMIDES
SOLUTIONS CONCENTREES DE POLYHYDROXYALKYLAMINES ET LEUR UTILISATION, NOTAMMENT POUR LA PRODUCTION D'AMIDES D'ACIDE POLYHYDROXYALKYL CARBOXYLIQUE

(30) Priorität: 30.05.1995 DE 19519705
(43) Veröffentlichungstag der Anmeldung: 08.04.1998
(73) Patentinhaber: Clariant GmbH, 65929 Frankfurt am Main (DE)
(72) Erfinder: PAPENFUHS, Bernd, D-63179 Obertshausen (DE)
(86) Internationale Anmeldenummer: EP9602231
(87) Internationale Veröffentlichungsnummer: WO9638404

(56) Entgegenhaltungen:
- DE-A- 2 349 278
- DE-A- 4 322 874
- US-A- 4 021 539

## Beschreibung

Die Erfindung betrifft konzentrierte Lösungen von Polyhydroxyalkylaminen in Wasser, Alkoholen oder in Wasser/Alkohol-Gemischen als Lösungsmittel und die Verwendung dieser Lösungen zur Herstellung von Polyhydroxyalkylcarbonsäureamiden.

Polyhydroxyalkylamine sind wertvolle Verbindungen zur Bereitung von Kosmetikmitteln, vergleiche US-A-4 021 539. Sie stellen ferner vorteilhafte Ausgangsverbindungen dar zur Herstellung von Harnstoffderivaten, vergleiche DE-A-23 49 278, und insbesondere zur Herstellung der entsprechenden Carbonsäureamide durch Acylierung (Amidierung) der Aminverbindung mit Fettsäure oder Fettsäurederivaten wie Fettsäurealkylestern, vergleiche DE-A-43 22 874. Zu den Polyhydroxyalkylaminen zählen insbesondere die Aminozucker, die im Falle von Glucoseresten als Glucamine und im Falle von anderen Zuckerresten als Glycamine bezeichnet werden. Entsprechend gelten für die resultierenden Carbonsäureamide die Bezeichnungen Glucamide beziehungsweise Glycamide. Im folgenden werden die in Rede stehenden Verbindungen - nur der Kürze wegen - als Glucamine und Glucamide bezeichnet.

Glucamine werden durch katalytische reduktive Aminierung von Glucose mit Aminen hergestellt und in der Regel als wäßrige, alkoholische oder wäßrig/alkoholische Lösungen insbesondere zur Herstellung der entsprechenden besonders oberflächenaktiven Glucamide angeboten. Zur Glucamidherstellung werden die Glucamine in Form einer konzentrierten alkoholischen Lösung und bevorzugt in Form einer Glucaminschmelze eingesetzt, vergleiche die obengenannte DE-A-43 22 874. In beiden Fällen sind also mehr oder weniger konzentrierte Glucaminlösungen erwünscht. So können konzentrierte alkoholische Lösungen bereits als solche eingesetzt werden. Will man die Glucaminverbindung in Form einer Schmelze einsetzen, so ist im Falle von konzentrierten Lösungen ein geringerer Aufwand an Einrichtungen und Energie zur Entfernung des Lösungsmittels erforderlich als im Falle von verdünnten Lösungen. Ein Transport der Lösungen verursacht ebenfalls je nach Konzentration an Glucamin mehr oder weniger hohe Kosten. Ein Transport in Schmelze ist problematisch aufgrund der mangelhaften Thermostabilität von Glucaminen ganz allgemein. Daraus folgt, daß solche Glucamine besonders erwünscht wären, die in Wasser, Alkohol oder Waser/Alkohol-Mischungen gut löslich sind. Mit solchen Glucaminverbindungen könnte man konzentrierte Lösungen bereiten, womit ein besonders vorteilhaftes Produkt zur Herstellung von Glucamiden zur Verfügung stünde.

Überraschenderweise wurde gefunden, daß unter den zahlreichen Glucaminverbindungen jene aus der Gruppe der Dialkylaminopropyl-glucamine eine besonders hohe Löslichkeit in Wasser, Alkoholen, insbesondere C₁ bis C₃-Alkoholen, und in Mischungen aus Wasser und Alkoholen aufweisen.

Gefunden wurden demnach konzentrierte wäßrige, alkoholische und wäßrig/alkoholische Lösungen von mindestens einem Dialkylaminopropyl-glucamin der nachstehenden Formel 1

Z-NH-CH₂CH₂CH₂-NR¹R² (1)

worin R¹ und R², gleich oder verschieden, C₁ bis C₄-Alkyl oder Hydroxyalkyl mit 2 bis 4 Kohlenstoffatomen und Z einen linearen Polyhydroxykohlenwasserstoffrest mit mindestens drei gegebenenfalls oxalkylierten OH-Gruppen bedeuten, bestehend im wesentlichen aus a) 45 bis 90 Gew.-% von mindestens einer Verbindung der Formel 1 und b) 10 bis 55 Gew.-% von Wasser, von mindestens einem C₁ bis C₃-Alkohol oder von Wasser und mindestens einem C₁ bis C₃-Alkohol als Lösungsmittel, Gewichtsprozente bezogen auf die Lösung.

Bevorzugt sind Verbindungen der Formel 1, wobei R¹ und R² Methyl sind und Z der Rest eines Zuckeralkohols ist, der sich von einem reduzierenden Mono- oder Disaccharid, insbesondere von Glucose, ableitet.

Bevorzugte erfindungsgemäße Lösungen bestehen im wesentlichen aus a) 55 bis 80 Gew.-%, insbesondere 60 bis 70 Gew.-%, von mindestens einer Verbindung der Formel 1 und b) 20 bis 45 Gew.-%, insbesondere 30 bis 40 Gew.%, von Wasser, von mindestens einem C₁ bis C₃-Alkohol oder von Wasser und mindestens einem C₁ bis C₃-Alkohol als Lösungsmittel, Gewichtsprozente bezogen auf die Lösung.

Als C₁ bis C₃-Alkohole sind Methanol, Ethanol, Isopropanol und/oder 1,2-Propandiol bevorzugt. Das Volumenverhältnis im Lösungsmittel Wasser/Alkohol kann in weiten Grenzen variieren. So können Wasser/Alkohol-Mischungen eingesetzt werden, die vorwiegend aus Wasser oder vorwiegend aus Alkohol bestehen. Ein bevorzugtes Volumenverhältnis der Wasser/Alkohol-Mischungen liegt bei 1 : 2 bis 2 : 1, wobei 1 : 1 besonders bevorzugt ist. Von den genannten Lösungsmitteln sind Wasser und Wasser/Alkohol-Gemische bevorzugt.

Die erfindungsgemäßen Lösungen erscheinen dem menschlichen Auge bei Temperaturen oberhalb von etwa 15 °C klar und niederschlagsfrei. Die erfindungsgemäß vorgeschlagenen N, N-Dialkyl-N'-polyhydroxyalkylpropylendiamine der Formel 1 sind also bei Raumtemperatur und ebenso bei höheren Temperaturen in den genannten Lösungsmitteln gut löslich. Ein zweckmäßiger Temperaturbereich auch im Hinblick auf Transport und Lagerung der konzentrierten Lösungen liegt bei etwa 15 bis 70 °C. Die Löslichkeit bei Raumtemperatur ist in Wasser und in Wasser/Alkohol-Mischungen etwas besser als in Alkohol allein. Bei höheren Temperaturen ist die Löslichkeit in allen drei Lösungsmitteln praktisch gleich gut. So bestehen erfindungsgemäße Lösungen bei Raumtemperatur zum Beispiel im wesentlichen aus a) 55 bis 70 Gew.-% von mindestens einer Verbindung der Formel 1 und b) 30 bis 45 Gew.-% Wasser oder Wasser/Alkohol-Mischung, Gewichtsprozente bezogen auf die Lösung. Bei höheren Temperaturen, zum Beispiel bei 50 bis 70 °C, sind hochkonzentrierte Lösungen möglich, wobei auch Alkohol allein das Lösungsmittel sein kann. Solche Lösungen bestehen beispielsweise im wesentlichen aus a) 55 bis 90 Gew.-% von mindestens einer Verbindung der Formel 1 und b) 10 bis 45 Gew.-% Lösungsmittel, Gewichtsprozente bezogen auf die Lösung.

Die Herstellung der erfindungsgemäßen konzentrierten Glucaminlösungen erfolgt vorzugsweise im Rahmen der oben erwähnten reduktiven Aminierung. Dabei werden Zuckerverbindungen entsprechend dem Rest Z in Formel 1 mit einem N,N-Dialkyl-alkylendiamin der Formel 2

H₂N-(CH₂)₃-NR¹R² (2)

worin R¹ und R² die genannten Bedeutungen haben, in den genannten Lösungsmitteln Wasser, Alkohol oder Wasser und Alkohol und in Gegenwart von Hydrierungskatalysatoren reduktiv zur Glucaminverbindung der Formel 1 aminiert. Das Molverhältnis von Zuckerverbindung zu Amin liegt bei etwa 1 : 1 bis 1,2. Das Lösungsmittel wird in einer Menge von etwa 5 bis 50 Gew.-%, bezogen auf gebildete Glucaminverbindung, eingesetzt. Als Lösungsmittel sind Wasser und die genannten Wasser/Alkohol-Mischungen bevorzugt. Als Katalysatoren können die üblichen Hydrierungskatalysatoren wie Palladium auf Aktivkohle, Kupferchromit und insbesondere Raney-Nickel in einer Menge von im allgemeinen 0,01 bis 3 Gew.-%, vorzugsweise 0,1 bis 1 Gew.-%, bezogen auf die zu aminierende Zuckerverbindung, eingesetzt werden. Die reduktive Aminierungsreaktion wird bei einer Temperatur von 40 bis 150 °C, vorzugsweise 50 bis 120 °C, und bei einem Wasserstoffdruck von 10 bis 200 bar, vorzugsweise 20 bis 100 bar, durchgeführt. Von den erhaltenen Lösungen mit der gebildeten Glucaminverbindung kann der Katalysator zum Beispiel durch Filtration abgetrennt werden. Aufgrund der guten Löslichkeit des in Rede stehenden Glucamins können verdünnte Reaktionslösungen aufkonzentriert werden, zum Beispiel einfach durch Abdampfen oder Abdestillieren des Lösungsmittels.

Die erfindungsgemäß vorgeschlagenen N-(3-Dialkylamino)propyl-N-polyhydroxyalkylamine weisen - wie bereits hervorgehoben - eine unerwartet hohe Löslichkeit in den beschriebenen Lösungsmitteln auf. Aufgrund dieser gegenüber anderen Glucaminen hervorragenden Eigenschaft eignen sie sich insbesondere zur Herstellung der entsprechenden Amidverbindungen. Die Erfindung bezieht sich deshalb auch auf die Verwendung der beschriebenen Lösungen mit den N-funktionalisierten Glucaminen zur Herstellung von Polyhydroxyalkylcarbonsäureamiden der Formel 3 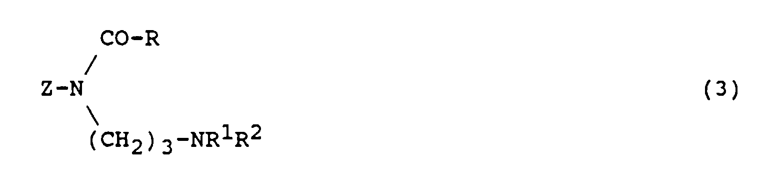 worin R ein aliphatischer Rest mit 5 bis 23 Kohlenstoffatomen ist, vorzugsweise ein Fettalkylrest mit 7 bis 17 Kohlenstoffatomen, und R¹, R² und Z die angegebenen Bedeutungen haben. Die Herstellung dieser N-(3-Dialkylamino)-propyl-N-polyhydroxyalkylcarbonsäureamide, insbesondere der entsprechenden Glucamide, kann nach dem Verfahren erfolgen, das in der oben erwähnten Druckschrift DE-A-43 22 874 beschrieben ist. Danach wird man von den erfindungsgemäßen konzentrierten oder hochkonzentrierten Lösungen das restliche Lösungsmittel entfernen und das Glucamin in Form einer Schmelze mit dem Amidierungsmittel Fettsäure oder Fettsäurederivat, vorzugsweise Fettsäure-C₁ bis C₄-alkylester, umsetzen zur Bildung der entsprechenden N-funktionalisierten Glucamide. Diese bevorzugte Umsetzung unter Einsatz der Glucaminverbindung in Form einer Schmelze wird bei einer Temperatur von etwa 60 bis 130 °C und in Gegenwart eines alkalischen Katalysators durchgeführt.

Sie ist in DE-A-43 22 874 im einzelnen beschrieben, die hier miteinbezogen wird.

Zum Polyhydroxyalkylrest Z in Formel 1 sei noch folgendes gesagt: Dieser Rest stammt, wie oben bereits erwähnt, vorzugsweise von Polyhydroxyalkylverbindungen aus der Gruppe der reduzierenden Zucker oder reduzierenden Zuckerderivate. Bevorzugte reduzierende Zuckerverbindungen sind die Monosaccharide, vorzugsweise Pentosen und Hexosen, und die Oligosaccharide, vorzugsweise Disaccharide und gegebenenfalls auch Trisaccharide. Beispiele für Monosaccharide sind Glucose, Galaktose, Mannose und Talose als Hexosen und Arabinose, Ribose und Xylose als Pentosen. Von den Monosacchariden sind die Hexosen bevorzugt. Beispiele für Oligosaccharide (Polysaccharide) sind Lactose, Maltose, Maltotriose und dergleichen. Besonders bevorzugte Polyhydroxyalkylreste stammen von reduzierenden Hexosen, insbesondere von Glucose (Sorbitylrest).

Die Erfindung wird nun anhand eines Berichtes über die Löslichkeit verschiedener Glucamine noch näher erläutert.

Es wurde die Löslichkeit von N-(3-Dimethylamino)propyl-glucamin gemäß Formel 1 und von analogen N-substituierten Glucaminen, nämlich N-Methyl-glucamin, N-Butyl-glucamin und N-Hexyl-glucamin, in Wasser, in einer Mischung aus Wasser und Isopropanol im Volumenverhältnis von 1 : 1 und in Methanol getestet. Die bezüglich Löslichkeit zu vergleichenden Glucaminverbindungen wurden in Konzentrationen von 10 bis 70 Gew.-% unter Rühren in den genannten Lösungsmitteln bei 60 °C aufgenommen und anschließend bei 60 °C und bei Raumtemperatur (20 °C) 5 Tage lang gelagert. Die Ergebnisse dieser Lagerversuche sind in der nachstehenden Tabelle zusammengefaßt, in der "+" dünnflüssige, lagerstabile und dem menschlichen Auge klar und niederschlagsfrei erscheinende Lösungen bedeutet und "-" die Bildung von Eintrübungen, Ausfällungen oder sogar Erstarrung. Ferner bedeuten die Abkürzungen "RT" Raumtemperatur und "DMAP-Glucamin" N-Dimethylaminopropyl-glucamin gemäß Formel 1. Wie die Tabelle zeigt, sind die Unterschiede in der Löslichkeit in Methanol als Lösungsmittel am stärksten ausgeprägt. Auch in Wasser sind N-Butyl-glucamin und N-Hexyl-glucamin praktisch unlöslich und das kürzerkettige N-Methyl-glucamin kann nur bei höherer Temperatur (60 °C) hochkonzentriert solvatisiert werden. Das erfindungsgemäß vorgeschlagene N-Dimethylaminopropyl-glucamin ist dagegen unter allen getesteten Bedingungen wesentlich besser löslich, so daß die beschriebenen konzentrierten bis hochkonzentrierten Lösungen möglich sind.

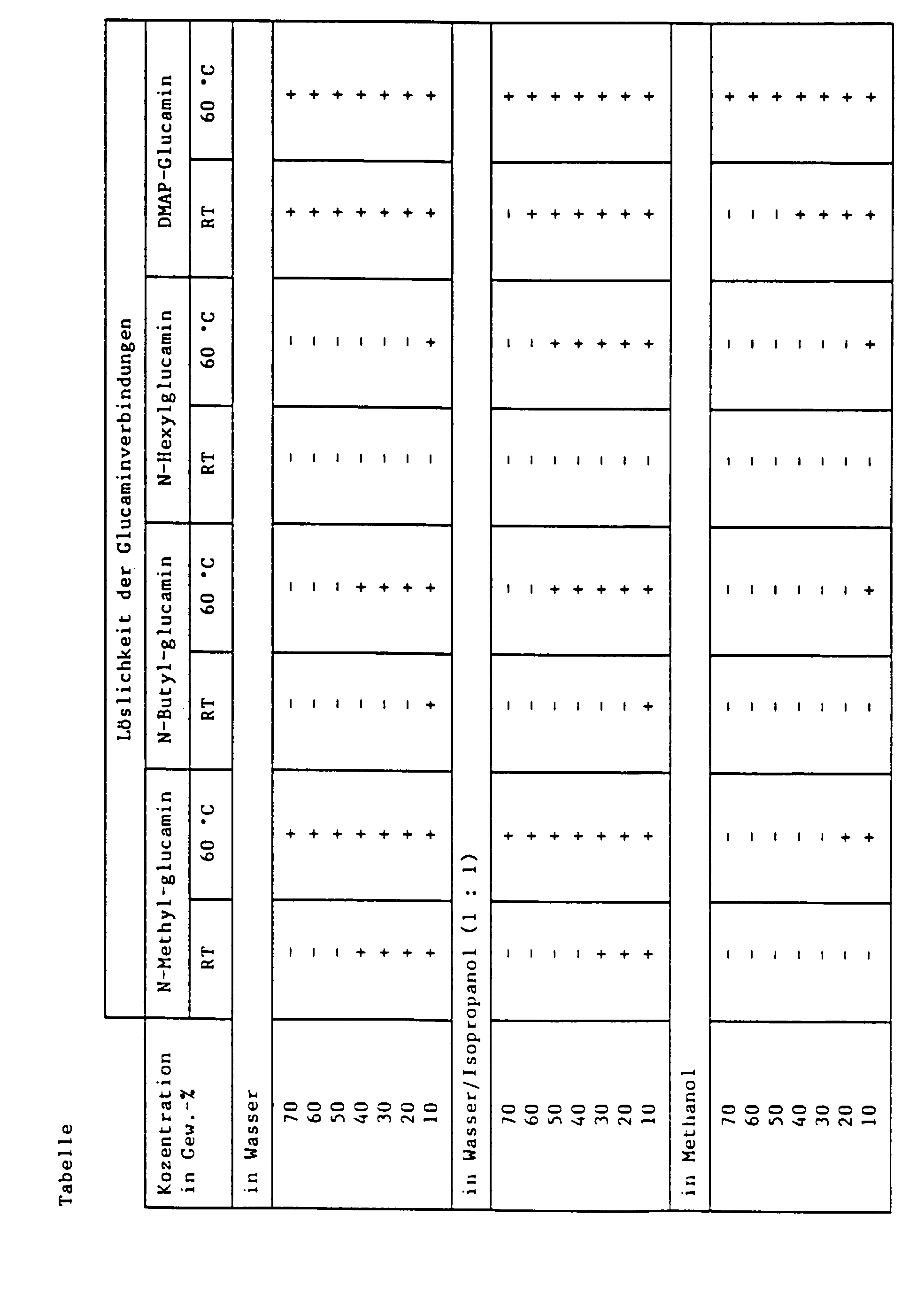

## Patentansprüche

1. Konzentrierte wäßrige, alkoholische und wäßrig/alkoholische Lösungen von mindestens einem Dialkylaminopropyl-glucamin der nachstehenden Formel 1
Z-NH-CH₂CH₂CH₂-NR¹R² (1)
worin R¹ und R², gleich oder verschieden, C₁ bis C₄-Alkyl oder Hydroxyalkyl mit 2 bis 4 Kohlenstoffatomen und Z einen linearen Polyhydroxykohlenwasserstoffrest mit mindestens drei gegebenenfalls oxalkylierten OH-Gruppen bedeuten, bestehend im wesentlichen aus a) 45 bis 90 Gew.-% von mindestens einer Verbindung der Formel 1 und b) 10 bis 55 Gew.-% von Wasser, von mindestens einem C₁ bis C₃-Alkohol oder von Wasser und mindestens einem C₁ bis C₃-Alkohol als Lösungsmittel, Gewichtsprozente bezogen auf die Lösung.

2. Lösungen nach Anspruch 1, bestehend im wesentlichen aus a) 55 bis 80 Gew.-% von mindestens einer Verbindung der Formel 1 und b) 20 bis 45 Gew.-% von Wasser, von mindestens einem C₁ bis C₃-Alkohol oder von Wasser und mindestens einem C₁ bis C₃-Alkohol als Lösungsmittel, Gewichtsprozente bezogen auf die Lösung.

3. Lösungen nach Anspruch 1, bestehend im wesentlichen aus a) 60 bis 70 Gew.-% von mindestens einer Verbindung der Formel 1 und b) 30 bis 40 Gew.-% von Wasser, von mindestens einem C₁ bis C₃-Alkohol oder von Wasser und mindestens einem C₁ bis C₃-Alkohol als Lösungsmittel, Gewichtsprozente bezogen auf die Lösung.

4. Lösungen nach einem oder mehreren der Ansprüche 1 bis 3, dadurch gekennzeichnet, daß das Lösungsmittel Wasser oder ein Wasser/Alkohol-Gemisch ist.

5. Lösungen nach einem oder mehreren der Ansprüche 1 bis 4, dadurch gekennzeichnet, daß der Alkohol Methanol, Ethanol, Isopropanol oder 1,2-Propandiol oder eine Mischung aus zwei oder mehreren dieser Alkohole ist.

6. Lösungen nach einem oder mehreren der Ansprüche 1 bis 5, dadurch gekennzeichnet, daß R¹ und R² Methyl sind und Z der Rest eines Zuckeralkohols ist, der sich von einem reduzierenden Mono- oder Disaccharid ableitet.

7. Lösungen nach einem oder mehreren der Ansprüche 1 bis 5, dadurch gekennzeichnet, daß R¹ und R² Methyl sind und Z der Sorbitylrest ist.

8. Verwendung der Lösungen nach Anspruch 1 zur Herstellung von Polyhydroxyalkylcarbonsäureamiden der nachstehenden Formel 3 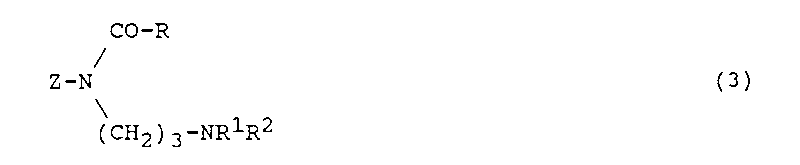 worin R ein aliphatischer Rest mit 5 bis 23 Kohlenstoffatomen ist und R¹, R² und Z die angegebenen Bedeutungen haben.

## Claims

1. A concentrated aqueous, alcoholic or aqueous/alcoholic solution of at least one dialkylaminopropylglucamine of the formula 1 below
Z-NH-CH₂CH₂CH₂-NR¹R² (1)
where R¹ and R² are identical or different and are C₁-C₄-alkyl or hydroxyalkyl having from 2 to 4 carbon atoms and Z is a linear polyhydroxyhydrocarbon radical having at least three OH groups which may, if desired, be alkoxylated, consisting essentially of a) from 45 to 90% by weight of at least one compound of the formula 1 and b) from 10 to 55% by weight of water, of at least one C₁-C₃-alcohol or of water and at least one C₁-C₃-alcohol as solvent, percentages by weight being based on the solution.

2. A solution as claimed in claim 1, consisting essentially of a) from 55 to 80% by weight of at least one compound of the formula 1 and b) from 20 to 45% by weight of water, of at least one C₁-C₃-alcohol or of water and at least one C₁-C₃-alcohol as solvent, percentages by weight being based on the solution.

3. A solution as claimed in claim 1, consisting essentially of a) from 60 to 70% by weight of at least one compound of the formula 1 and b) from 30 to 40% by weight of water, of at least one C₁-C₃-alcohol or of water and at least one C₁-C₃-alcohol as solvent, percentages by weight being based on the solution.

4. A solution as claimed in one or more of claims 1 to 3, wherein the solvent is water or a water/alcohol mixture.

5. A solution as claimed in one or more of claims 1 to 4, wherein the alcohol is methanol, ethanol, isopropanol or 1,2-propanediol or is a mixture of two or more of these alcohols.

6. A solution as claimed in one or more of claims 1 to 5, wherein R¹ and R² are methyl and Z is the radical of a sugar alcohol which is derived from a reducing monosaccharide or disaccharide.

7. A solution as claimed in one or more of claims 1 to 5, wherein R¹ and R² are methyl and Z is the sorbityl radical.

8. The use of the solutions as claimed in claim 1 for preparing polyhydroxyalkylcarboxamides of the formula 3 below 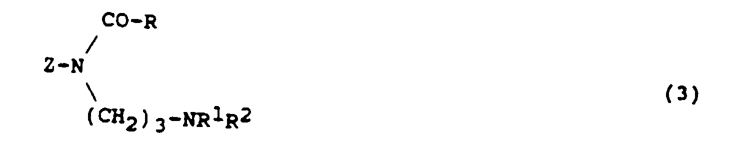 where R is an aliphatic radical having from 5 to 23 carbon atoms and R¹, R² and Z are as defined above.

## Revendications

1. Solutions aqueuses, alcooliques et aqueuses/alcooliques concentrées d'au moins une dialkylaminopropylglucamine ayant la formule suivante :
Z-NH-CH₂CH₂CH₂-NR¹R² (1)
dans laquelle R¹ et R², identiques ou différents, représentent des restes alkyle en C₁-C₄ ou hydroxyalkyle de 2 à 4 atomes de carbone, et Z représente un reste hydrocarboné polyhydroxylé linéaire contenant au moins 3 groupes OH éventuellement oxyalkylés, constituées essentiellement de
c) 45 à 90 % en masse d'au moins un composé de formule 1 et de
d) 10 à 55 % en masse d'eau, d'au moins un alcool en C₁-C₃ ou d'eau et d'au moins un alcool en C₁-C₃ comme solvant,
les pourcentages en masse se rapportant à la solution.

2. Solutions selon la revendication 1, constituées essentiellement de
c) 55 à 80 % en masse d'au moins un composé de formule 1 et de
d) 20 à 45 % en masse d'eau, d'au moins un alcool en C₁-C₃ ou d'eau et d'au moins un alcool en C₁-C₃ comme solvant,
les pourcentages en masse se rapportant à la solution.

3. Solutions selon la revendication 1, constituées essentiellement de
a) 60 à 70 % en masse d'au moins un composé de formule 1 et de
b) 30 à 40 % en masse d'eau, d'au moins un alcool en C₁-C₃ ou d'eau et d'au moins un alcool en C₁-C₃ comme solvant,
les pourcentages en masse se rapportant à la solution.

4. Solutions selon l'une ou plusieurs des revendications 1 à 3, caractérisées en ce que le solvant est de l'eau ou un mélange eau/alcool.

5. Solutions selon l'une ou plusieurs des revendications 1 à 4, caractérisées en ce que l'alcool est le méthanol, l'éthanol, l'isopropanol ou le 1,2-propanediol ou un mélange de deux ou plusieurs de ces alcools.

6. Solutions selon l'une ou plusieurs des revendications 1 à 5, caractérisées en ce que R¹ et R² sont des restes méthyle et Z est le reste d'un alcool de sucre dérivant d'un mono- ou disaccharide réducteur.

7. Solutions selon l'une ou plusieurs des revendications 1 à 5, caractérisées en ce que R¹ et R² sont des restes méthyle et Z est le reste sorbityle.

8. Utilisation des solutions selon la revendication 1 pour la préparation d'amides d'acides polyhydroxyalkylcarboxyliques ayant la formule 3 suivante : 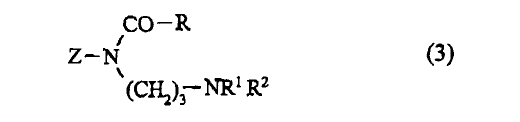 dans laquelle R est un reste aliphatique de 5 à 23 atomes de carbone et R¹, R² et Z ont les significations indiquées.
